# EUROPEAN PATENT APPLICATION

(11) **EP 0 815 816 A1**
(43) Date of publication of application: **07.01.1998**
(21) Application number: 96110573.1
(22) Date of filing: 01.07.1996
(51) Int. Cl.: A61F 13/15

(54) **Disposable absorbent article having expanding and shrinking elements and being capable of self-shaping in use**

(71) Applicant: THE PROCTER & GAMBLE COMPANY, Cincinnati, Ohio 45202 (US)
(72) Inventor: Carlucci, Giovanni, 66100 Chieti (IT); Tamburro, Maurizio, 65100 Pescara (IT); Giorgini, Gennaro, 64026 Roseto (IT)
(74) Representative: Hirsch, Uwe Thomas

(57) **Abstract**

A disposable absorbent article which is substantially flat prior to use for wearing adjacent a body discharge area having a longitudinal centreline and a lateral centreline orthogonal thereto and defining longitudinal and lateral directions respectively. The disposable absorbent article comprises a liquid pervious topsheet, a backsheet joined to said topsheet and an absorbent core intermediate the backsheet and the topsheet. The absorbent core comprises means for expanding the article into a tridimensional structure while being worn by a user. The article further comprises shrinkable means for inducing forming or bending of the article in combination with the expanding means. Both expanding and shrinkable means are activatable by body fluids.

## Description

### FIELD OF THE INVENTION

The present invention relates to disposable absorbent articles. Disposable absorbent articles are considered to be absorbent devices designed to be worn externally of the body by a user and to receive fluids discharged from the body. In particular the present invention relates to substantially flat disposable absorbent sanitary napkins, catamenials, incontinence inserts, pantiliners and diapers comprising expanding means for expanding the article into a tridimensional structure while being worn by a user. The absorbent article comprises shrinkable means for further inducing forming or bending of the absorbent article in combination with the expanding means, both expanding means and shrinkable means being activated by body fluids.

### BACKGROUND OF THE INVENTION

In their basic form, disposable absorbent articles comprise an absorbent core interposed between a pervious body-contacting element (alternatively referred to as a topsheet or an overwrap) and an impervious protective barrier (alternatively referred to as a backsheet). The absorbent element is, of course, intended to receive and contain the fluids discharged from the body. The body-contacting element is intended to provide comfortable and dry-feeling contact with body surfaces while allowing free passage of fluids therethrough into the absorbent element. The protective barrier is intended to prevent the fluids which are expelled or which escape from the absorbent element from soiling the user's garments.

In addition to the three functional elements mentioned above, disposable absorbent articles are generally provided with means for supporting the device adjacent the user's crotch area, even as the user moves, where it can most effectively perform its intended function. Typically, absorbent articles as sanitary napkins are provided with an adhesive attachment means for securing the device to the inner crotch area of the user's undergarments.

Improved fluid interception can occur if the absorbent article is in close contact with the body of the wearer.

While previously known absorbent articles perform their intended function, each conventional design can be further improved in one or more of absorbency of body fluids, protection of the user's garments from soiling, and/or physical comfort to the user.

With respect to disposable sanitary napkins several attempts have been made in the art to improve body contact with the wearer, and hence absorb fluids upon discharge and thereby minimize soiling by providing a sanitary napkin having an anatomically shaped configuration, particularly including those that are raised upwardly or humped in their medial portions so as to be near or in contact with the pudendal region when worn.

On female users these sanitary napkins attempt to contact and absorb menses immediately as it leaves the vestibule.

Some articles have been also described in which an anatomically shaped configuration is provided during the wearing time, with the advantage of a better fit to the anatomy.

U.S. Patent No. 3,736,931 discloses a sanitary napkin having an outer non-compressed layer of fluid absorbent material and an inner core of highly compressed fluid absorbent material which is at least partially enclosed therein. The napkin preferably is V-shaped in cross section and is arch-shaped in its longitudinal direction by die compression. When the napkin is worn the fluid directs first into the inner compressed layer so as to cause it to swell and to expand the outer non-compressed layer in all directions, thereby adjusting itself to each wearer.

The sanitary napkin expands upon fluid absorption and may adjust itself to the user's anatomy, but since it is not flat prior to use it may be cumbersome to package and to handle.

According to U.S. Patent No. 3,512,530 a sanitary napkin is described in which a compressed regenerated cellulose sponge layer is combined to a larger fibrous cellulose layer to form a multiple ply absorbent core. The compressed regenerated cellulose sponge layer is positioned over the fibrous layer, and it is typically centered about it; it is intended as the primary absorbent element of the sanitary napkin, while the fibrous layer acts as a secondary or back up absorber.

The sanitary napkin may be therefore very thin prior to use, as compared to other sanitary products having the same absorbent capacity.

Although the compressed regenerated cellulose sponge layer is capable of expanding in Z-direction upon fluid absorption, the structure described is not particularly suitable to provide an effective body contact with the wearer's anatomy and might cause discomfort to the user.

In European patent application EP 96106721.2, filed on 29 April 1996, an absorbent article is described which comprises a sheet of compressed regenerated cellulose sponge for expanding the article into a tridimensional structure while being worn by a user, the sheet being activated by body fluids; the absorbent article further comprises a topsheet that is capable of expanding as the absorbent article expands.

The absorbent article is therefore capable of providing an anatomically shaped configuration for a closer body contact which is achieved during the use upon activation by absorbed body fluids, while it is comfortable for the wearer and easy to produce and to package. The performance of the absorbent article can be improved in terms of a better effectiveness in achieving a self conforming tridimensional structure even upon acquisition of very small amounts of fluid.

A further improvement has been found when the self-conforming absorbent article is provided with better anti-bunching capability.

It is an object of the present invention to provide an absorbent article capable of providing an anatomically shaped configuration for a closer body contact which is achieved during the use upon activation by absorbed body fluids, while it is comfortable for the wearer, easy to produce and to package, and capable of achieving a high degree of swelling combined with a better self-conforming capability even upon activation by small amounts of body fluids.

### SUMMARY OF THE INVENTION

The present invention relates to disposable absorbent articles for wearing adjacent a body discharge area, which are substantially flat prior to use. The substantially flat disposable absorbent article has a longitudinal centreline and a lateral centreline orthogonal thereto that define longitudinal and lateral directions respectively, and a Z-direction that is orthogonal to both of them. The disposable absorbent article comprises a liquid pervious topsheet, a backsheet, preferably liquid impervious, joined to said topsheet, and an absorbent core intermediate the topsheet and the backsheet. The term "substantially flat", as used herein, refers to articles that have their main extension in one plane in contrast to being shaped. The absorbent core comprises expanding means for expanding the article into a tridimensional structure during the wearing time, the expanding means being activated by body fluids. The absorbent article also comprises means for further inducing forming or bending of the article in combination with the expanding means, this further means being capable of shrinking upon activation of body fluids.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a top plan view of one embodiment of a sanitary napkin according to the present invention;
FIG. 2 is a cross-sectional view of the sanitary napkin shown in FIG. 1 as taken along a section line corresponding to the longitudinal centreline O-O;
FIG. 3 is a cross-sectional view similar to that of FIG. 2, showing the sanitary napkin after a first extension into a tridimensional structure upon activation during wear;
FIG. 4 is a cross-sectional view similar to that of FIG. 2, showing the sanitary napkin after a further extension into a tridimensional structure upon activation during wear;
FIG. 5 is a cross-sectional view, taken along a section line corresponding to the transverse centreline A-A, of another embodiment of a sanitary napkin according to the present invention;
FIG. 6 is a cross-sectional view showing the sanitary napkin of FIG. 5 expanded in the tridimensional structure after activation during wear;
FIG. 7 is a cross-sectional view, taken along a section line corresponding to the transverse centreline A-A, of another embodiment of a sanitary napkin according to the present invention;
FIG. 8 is a cross-sectional view showing the sanitary napkin of FIG. 7 after a first extension into the tridimensional structure upon activation during wear.
FIG. 9 is a cross-sectional view showing the sanitary napkin of FIG. 7 after activation of the further shrinkable means during wear.

### DETAILED DESCRIPTION OF THE INVENTION

This invention relates to a disposable absorbent article which exhibits absorbency for bodily fluids, the protection of the user's garments from soiling, improved physical comfort to the user, and which is easy to produce and to package. The disposable absorbent article is described below by reference to a sanitary napkin or catamenial. The term "sanitary napkin", as used herein, refers to an article which is worn by females adjacent to the pudendal region and which is intended to absorb and contain the various body fluids which are discharged from the body (e.g., vaginal discharges, menses, and/or urine) and which is intended to be discarded after a single use. The disposable absorbent article is substantially flat prior to use.

The term "substantially flat", as used herein, refers to articles that have their main extension in one plane in contrast to being shaped. In a preferred embodiment a substantially flat article will have an absorbent core of constant thickness or, at least, will have an absorbent core that is not shaped in a direction which is orthogonal to the absorbent core itself. This does not exclude a general curvature of the absorbent core. It will be apparent to the man skilled in the art to which extent products can deviate from absolute flat shape and still benefit from the during the use shaping according to the present invention.

Sanitary napkins with longitudinal side cuffs, which may be optionally elasticated, and sanitary napkins with a moderate curvature are therefore within the scope of the present invention, provided that their absorbent core is not shaped prior to use in a direction that is orthogonal to the absorbent core itself.

The term "use", as used herein, refers to the period of time that starts when the absorbent article is actually put in contact with the anatomy of the user.

The terms "joined" or "affixed", as used herein, encompasses configurations whereby a first member is directly connected to a second member and configurations whereby a first member is indirectly connected to a second member by connecting the first member to intermediate members which in turn are connected to the second member.

Interlabial devices which reside partially within and partially external of the wearer's vestibule are also within the scope of this invention. As used herein, the term "pudendal" refers to the externally visible female genitalia and is limited to the labia majora, the labia minora, the clitoris, and the vestibule.

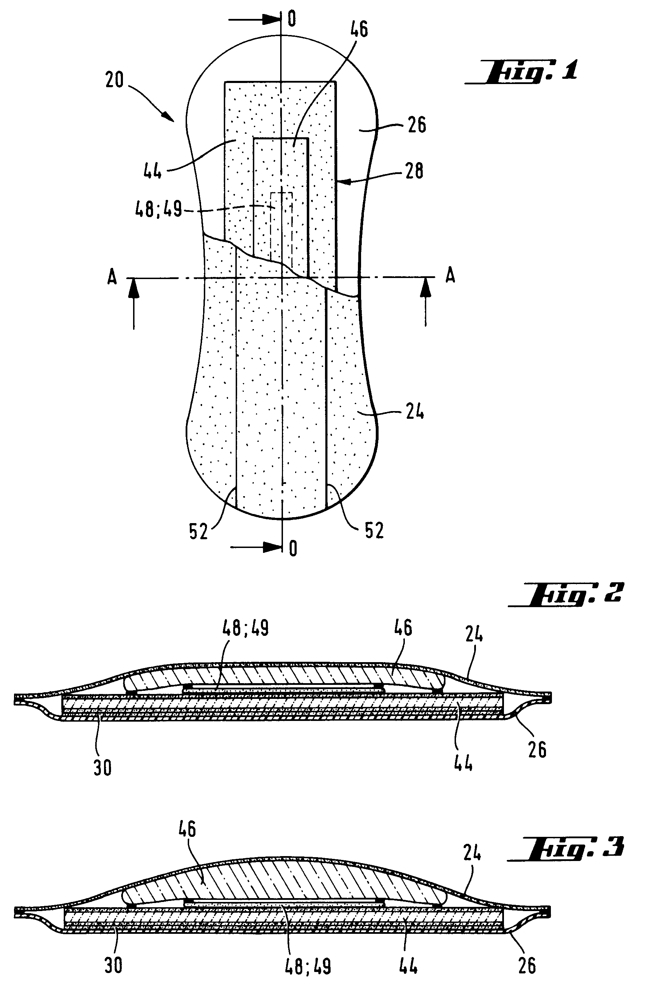

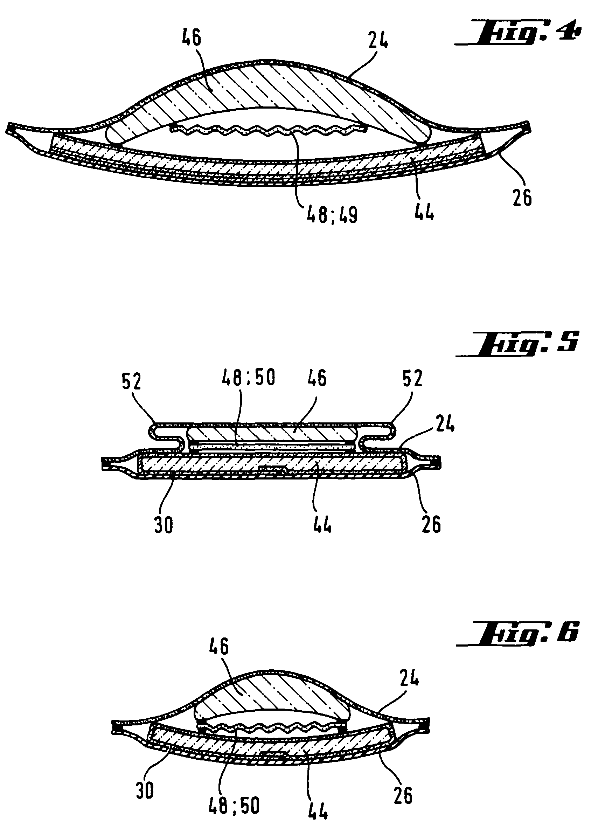

In FIGS. 1 and 2, one preferred embodiment of a sanitary napkin 20 of the present invention is shown. Fig. 1 is a plan view of the sanitary napkin 20 of the present invention in its flat-out state prior to use with portions of the structure being cut-away to more clearly show the construction of the sanitary napkin 20 and with the portion of the sanitary napkin 20 which faces or contacts the wearer oriented towards the viewer. As shown in FIGS. 1 and 2, the sanitary napkin 20 preferably comprises a liquid pervious topsheet 24, a liquid impervious backsheet 26 joined to the topsheet 24, and an absorbent core 28 intermediate the topsheet 24 and the backsheet 26, which comprises an expanding layer 46 as expanding means for expanding the sanitary napkin into a tridimensional structure while being worn by a user; the sanitary napkin 20 also comprises means 48 for further inducing forming or bending of the sanitary napkin, preferably into a tridimensional structure, in combination with the expanding means 46; means 48 is a shrinkable means in that it comprises means that are capable of shrinking upon activation by body fluids.

In a preferred embodiment of the present invention illustrated in FIGS. 1 to 4, the absorbent core 28 comprises the expanding layer 46 and a separate, substantially non expanding absorbent element 44 joined together and in face to face relationship to each other, the expanding layer 46 being positioned between the topsheet 24 and the absorbent element 44.

The absorbent element 44 and the expanding layer 46 may be associated in any suitable manner to form the absorbent core 28. Suitable manners include, but are not limited to, associating the absorbent element 44 and the expanding layer 46 with adhesives such as by spray-gluing or by applying lines or spots of adhesive between them. Alternatively, or additionally, the association between the layers may be achieved by fibre entanglement or by a plurality of discrete fusion bonds.

Alternatively, the expanding layer 46 may constitute the entire absorbent core 28.

The absorbent capacity of the absorbent core 28 should be compatible with the intended body fluid loading for the sanitary napkin 20. Further, the overall absorbent capacity of the absorbent core 28 may be varied to accommodate wearers ranging in the expected amount of body fluid volume. For instance, a different absorbent capacity may be utilized for sanitary napkins intended for day time use as compared with those intended for night time use, or for sanitary napkins intended for use by teenage females as compared with those intended by more mature women.

The sanitary napkin 20 has two surfaces, a body contacting or facing surface, and a garment facing or contacting surface. The absorbent core 28 has corresponding body facing and garment facing surfaces. The sanitary napkin 20 has two centrelines, a longitudinal centreline O-O and a transverse centreline A-A orthogonal thereto. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral", as used herein, are interchangeable, and refer to a line, axis, or direction which lies within the plane of the sanitary napkin 20 and is generally perpendicular to the longitudinal direction. The Z-direction is orthogonal to both the longitudinal and lateral directions of the sanitary napkin 20 and extends outwardly from the plane of the sanitary napkin 20, which is defined by the longitudinal centreline O-O and the lateral centreline A-A. The term "longitudinally oriented" refers to a direction ±45 degrees of the longitudinal direction in the plane of the sanitary napkin 20; the term "laterally oriented" refers similarly to any other direction in the plane of the sanitary napkin 20.

The long edges of the sanitary napkin 20, which are aligned with the longitudinal centreline O-O, are the longitudinal side margins of the sanitary napkin 20. The ends of the sanitary napkin 20 joining the longitudinal side margins are the transverse ends of the sanitary napkin 20. Collectively the longitudinal side margins and transverse ends of the sanitary napkin 20 define its periphery. Similarly, the absorbent core 28 of the sanitary napkin 20 has a periphery defined by alternatively disposed longitudinal side margins and transverse ends.

Tridimensional structures of the sanitary napkin 20 are those in which the sanitary napkin structure is caused to extend, at least partially, in the Z-direction, in order to more closely conform to the user's anatomy. Said extension in the Z-direction can be provided by the expansion of an expanding means in combination with the forming or bending of the structure achieved with a shrinking means. It is preferred that the activation by absorbed body fluids of both the expanding and the shrinkable means takes place substantially at the same time during the use of the sanitary napkin 20, but this can also be achieved at slightly different times. The extension preferably takes place in a direction that goes from the garment facing surface towards the body facing surface of the sanitary napkin 20, but an extension in Z-direction can also comprise a deformation of the structure of the sanitary napkin 20 in the opposite direction, i.e. from the body facing surface towards the garment facing surface. Particularly preferred are tridimensional structures with a convex upward configuration that are inclusive of, but not limited to, inverted U-shapes or inverted V-shapes, with "convex upward configuration" being meant a structure of the sanitary napkin 20 that is convex on its body facing surface. With these configurations the cross-sectional contour of the central portion of the sanitary napkin more closely matches the labia of the typical wearer.

The topsheet 24 is compliant, soft feeling, and non-irritating to the wearer's skin. Further, the topsheet 24 is liquid pervious, permitting liquid to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibres (e.g., polymeric fibres such as polyester, polypropylene, or polyethylene fibres); or from a combination of natural and synthetic fibres.

A preferred topsheet comprises an apertured formed film. Apertured formed films are preferred for the topsheet because they are pervious to body fluids and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer.

Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. A preferred topsheet for the absorbent article of the present invention is a formed film described in one or more of the above patents and marketed on sanitary napkins by The Procter & Gamble Company of Cincinnati, Ohio as "DRI-WEAVE".

In a preferred embodiment of the present invention, the body or exposed surface of the formed film topsheet is hydrophilic so as to help liquid transfer through the topsheet faster than if the body surface were not hydrophilic so as to diminish the likelihood that menstrual fluid will flow off the topsheet rather than flowing into and being absorbed by the absorbent core.

The topsheet of the present invention is preferably capable of expanding as the sanitary napkin 20 extends in a tridimensional structure upon absorption of body fluids. This may be achieved when the topsheet is made of a material that is intrinsically extensible under the forces exerted by the expanding means 46. In a preferred embodiment illustrated in FIGS. 1 the topsheet 24 is provided with two pleats or folds 52 symmetrically positioned at both sides of the longitudinal centreline O-O and substantially parallel to it. The details of the folds 52, which are not evident from FIGS. 1 to 4, are more clearly shown in FIG. 5 that illustrates an alternate embodiment of the sanitary napkin 20 of the present invention, which uses a topsheet 24 folded in the same way as in the embodiment of FIGS. 1 to 4. As better shown in FIG. 5 the topsheet 24 in each pleat or fold 52 is folded twice on itself toward the longitudinal side margins of the sanitary napkin 20. A single pleat or fold or, alternatively, more than two folds may be also comprised in the topsheet 24 without departing from the scope of the present invention; the pleats or folds may be generally longitudinally or laterally oriented.

The backsheet 26 is impervious to liquids (e.g., menses and/or urine) and is preferably manufactured from a thin plastic film, although other flexible liquid impervious materials can also be used. In use, the backsheet 26 is interposed between the absorbent core 28 and the user's undergarments. The function of the backsheet 26 is to prevent exudates which may be expelled from or which inadvertently bypass the absorbent core 28 from contacting and soiling the user's undergarments. The backsheet 26 can thus comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm to about 0.015 mm. The backsheet 26 is preferably embossed and/or matte finished to provide a more clothlike appearance.

In a preferred embodiment of the present invention the sanitary napkin 20 is also provided with a panty fastening means, not shown in the figures for clarity, which provides means to attach the article to an undergarment. For example the panty fastening means may comprise a mechanical fastener such as hook and loop fasteners such as marketed under the tradename VELCRO, snaps or holders. Alternatively, the sanitary napkin 20 is fastened to the undergarment by means of panty fastening adhesive on the backsheet 26. The panty fastening adhesive provides a means for securing the sanitary napkin 20 to the panty and preferably a means for securing the sanitary napkin 20 when soiled to the fold and wrap package for convenient disposal. Typically, at least a portion of the garment facing surface of the backsheet 26 is coated with adhesive to form the panty fastening adhesive. Any adhesive or glue used in the art for such purposes can be used for the panty fastening adhesive herein. Pressure sensitive adhesives are most preferred. Suitable adhesives include Century A-305-IV manufactured by the Century Adhesives Corporation of Columbus, Ohio, and Instant LOK 34-2823 manufactured by the National Starch and Chemical Company of Bridgewater, New Jersey, 3 Sigma 3153 manufactured by 3 Sigma and Fuller H-2238ZP manufactured by the H.B. Fuller Co.

The panty fastening adhesive is typically applied to the backsheet by slot coating or spraying in various distribution patterns, such as e.g. continuous or discontinuous strips, intermittent dots, random spiral patterns.

The panty fastening adhesive is typically covered with a removable release paper or film in order to prevent the adhesive from drying out or adhering to another surface other than the panty prior to use. Any commercially available release paper or film may be used. Suitable examples include BL 30MG-A SILOX EI/O and BL 30 MG-A SILOX 4 P/O available from Akrosil Corporation.

If present, as illustrated in FIGS. 1 to 4, the substantially non expanding absorbent element 44 of the absorbent core 28 may be any absorbent means which is generally compressible, resilient, non-irritating to the wearer's skin and capable of absorbing and containing body fluids. The absorbent element 44 may be manufactured from a wide variety of liquid absorbent materials commonly used in disposable sanitary napkins, and other disposable absorbent articles. Examples of suitable absorbent materials include comminuted wood pulp (which is generally referred to as airfelt), creped cellulose wadding, modified cross-linked cellulose fibres (such as those described in U.S. Patent No. 5,217,445 issued to Young, et al. on June 8, 1993), capillary channel fibres (that is, fibres having intra-fibre capillary channels such as those described in U.S. Patent No. 5,200,248 issued to Thompson, et a. on April 6, 1993), absorbent foams (such as those described in U.S. Patent No. 5,260,345, issued to DesMarais, et a. on November 9, 1993 and U.S. Patent No. 5,268,244 issued to DesMarais, et al. on December 7, 1993), thermally bonded airlaid materials (such as those material described in U.S. Patent Application Serial No. 08/141,156, entitled "Catamenial Absorbent Structures Having Thermally Bonded Layers For Improved Handling of Menstrual Fluids and Their Use In Catamenial Pads Having Improved Fit and Comfort" filed in the name of Richards, et al. on October 21, 1993), absorbent sponges, synthetic staple fibres, polymeric fibres, hydrogel-forming polymer gelling agents, peat moss, or any equivalent materials or combinations of materials. Suitable absorbent cores comprising foams are described in European Applications 0 598 833, 0 598 823 and 0 598 834.

In the embodiment illustrated in FIGS. 1 to 4, the absorbent element 44 of the absorbent core 28 comprises an absorbent layer 30 made of a thermally bonded airlaid material longitudinally folded twice on itself and comprising particles of absorbent gelling material therebetween, which are not shown for clarity.

As shown in FIGS. 1 and 2 the absorbent core 28 comprises an expanding layer 46 for expanding the sanitary napkin 20 into a tridimensional structure while the sanitary napkin 20 is being worn. In an embodiment illustrated in FIGS. 1 to 4 the extension and the shaping of the sanitary napkin 20 into the tridimensional structure is partially provided by the swelling, substantially in Z-direction, of an expanding layer 46 that constitutes the expanding means and that is activated during wear by the absorption of body fluids.

The expanding layer 46 that constitutes the expanding means can comprise any material that is capable of such swelling in order to shape the sanitary napkin 20 into a tridimensional structure.

After the absorption of body fluids and the subsequent swelling, the material of the expanding means 46 must be sort, compliant, conformable and resilient. It must be compressible such that it will deform under the relatively small forces that are experienced during normal use. In addition to be compressible, the material of the expanding means 46 must be flexible and conformable after swelling so it can provide improved fit through the topsheet 24 into and around the wearer's labia and perineum when the tridimensional structure is formed during the wearing time. The ability to follow the topography of the anatomy will provide intimate contact with the exposed genitalia of the female user. This helps provide better fluid transfer from the user into the expanding layer 46. While these characteristics of the expanding layer 46 allow for improved fit, they also cause the product to be both sort and comfortable for the wearer.

It is preferred that the expanding layer 46 forms at least part of the body facing surface of the absorbent core 28. In the embodiment illustrated in FIGS. 1 and 2 the expanding layer 46 that constitutes the expanding means is positioned over the body facing surface of the absorbent element 44, in face to face relationship with it; it is rectangular and preferably narrower and shorter than the absorbent element 44, as illustrated in FIG. 1, being centered about both the longitudinal and the transverse centrelines O-O and A-A. As an alternative, different shapes are also possible for the expanding layer 46, e.g. a layer with an hourglass shape.

The sanitary napkin 20 comprises shrinkable means 48 for further inducing forming or bending of the sanitary napkin 20 preferably into a tridimensional structure in combination with the expanding means, while the sanitary napkin 20 is being worn; the forming or bending of the sanitary napkin 20 is provided by the contraction or shrinking of the material that constitutes the shrinkable means 48 and that is activated during wear by body fluids.

The contracting material that constitutes the means 48 can be in form of film, stripes, yarns, fabric, or any other form, provided that this does not interfere with the contraction or shrinking itself, and provided that the physical characteristics and the form of the material are capable of imparting a contractive force sufficient to induce formation of the desired tridimensional structure in combination with the expanding means 46.

Suitable materials can be those that shrink upon contact with water or water-containing body fluids. Composite materials may also be used, which comprise an elastic material laminated in stretched condition to a non elastic, stiff material that dissolves or weakens upon contact with body fluids, so releasing the elastic tension of the elastic material that therefore is allowed to contract and to impart the desired tridimensional structure to the article during wear.

True shrinkable materials are generally capable of shrinking in the three directions upon activation, but since they are typically used as the shrinking means 48 in such a form that at least one dimension, as it is the case for films and stripes, or possibly two dimensions, as it is the case for yarns, are negligible with respect to the other(s), the shrinkage actually takes place only along the relevant direction(s) of the shrinking means. Shrinkage values are expressed in percentage of reduction of a given dimension upon complete activation, with respect to the original dimension before shrinking. Suitable shrinkage values may vary between 5% and 60%, preferably between 30% and 40%.

Referring to FIGS. 1 to 4, the sanitary napkin 20 comprises shrinkable means 48 for inducing forming or bending of the sanitary napkin 20 preferably into the tridimensional structure, in combination with the expanding layer 46, by shrinking upon activation by body fluids while the sanitary napkin 20 is being worn.

Shrinkable means 48 comprises a rectangular element 49 of a material that is capable of shrinking upon activation by body fluids; the element 49 spans the lateral centreline A-A of the sanitary napkin 20 and is generally aligned with the longitudinal centreline O-O. The element 49 is positioned between the expanding layer 46 and the substantially non expanding element 44 of the absorbent core 28, being centered about both the longitudinal centreline O-O and the lateral centreline A-A; therefore the corresponding longitudinal side margins and transverse ends of the substantially non expanding absorbent element 44 and of the expanding layer 46, and the corresponding longitudinal side margins and transverse ends of the shrinkable means 48 are respectively parallel to one another in the embodiment illustrated in FIGS. 1 and 2.

The length and the width of the element 49 are preferably lesser than the corresponding length and width of the expanding layer 46; in one preferred embodiment illustrated in FIGS. 1 to 4 the element 49 that constitutes the shrinkable means 48 is 110 mm long and 20 mm wide, while the corresponding lengths and widths of the expanding layer 46 and of the substantially non expanding absorbent element 44 are 125 mm, 30 mm, and 207 mm, 64 mm, respectively.

The element 49 is joined in any suitable manner, for example by stitching or glueing, and preferably at its transverse ends only, to the garment facing surface of the expanding layer 46, while the expanding layer 46 is preferably joined at its respective ends to the body facing surface of the substantially non expanding absorbent element 44; the joining can be achieved with any suitable means known in the art.

The extension of the sanitary napkin 20 into the Z-direction, in order to achieve a tridimensional structure that more closely conforms to the wearer's anatomy, is provided during the use by the expansion of the expanding layer 46 in combination with the shrinkage of the shrinkable means 48, upon activation by body fluids.

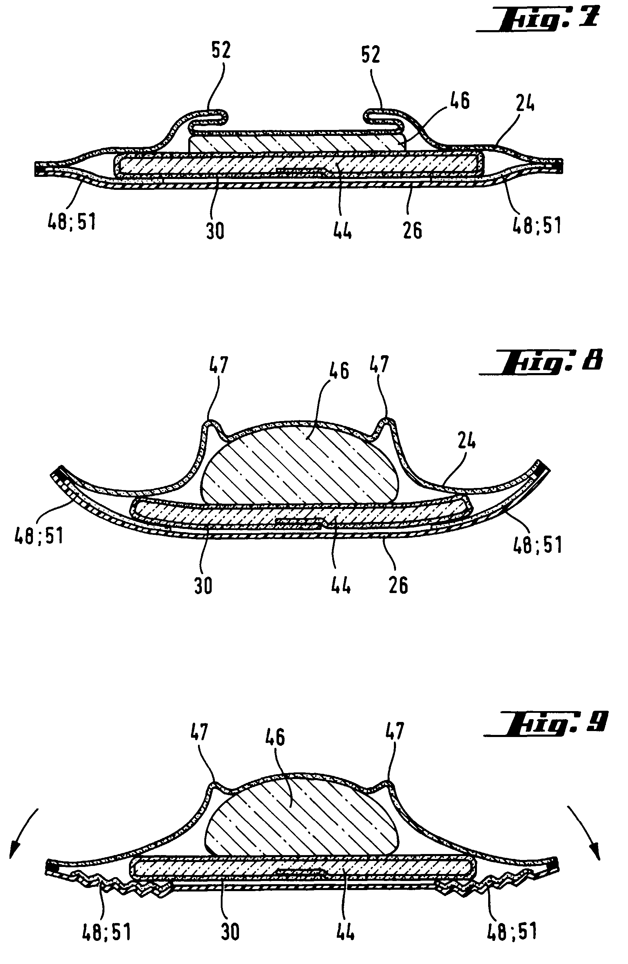

The combined effect of the expansion of the expanding layer 46 and of the shrinkage of the shrinkable means 48 upon activation of a first amount of body fluid is illustrated in FIGS. 3 and 4, while FIG. 2 shows the sanitary napkin 20 before the first fluid absorption, with the expanding layer 46 prior to swelling and with the shrinkable means 48 prior to shrinking.

FIG. 3 shows the sanitary napkin 20 after a first extension into a tridimensional structure upon activation of the expanding means 46 only by absorption of a first amount of body fluid in its central zone; this first release of fluid is rapidly acquired within the expanding means 46 and causes it to swell substantially in Z-direction. As soon as the fluid reaches the garment facing surface of the expanding means 46 it activates the shrinkable means 46 that is comprised between the expanding means 46 and the substantially non expanding absorbent element 44. The shrinkable means 48 is preferably capable of shrinking only along its longitudinal direction; upon activation by body fluid it begins to shrink and causes the already partially expanded expanding means 46, to which it is joined at its transverse ends, to achieve an arcuate shape with its convex side towards the body facing surface of the sanitary napkin 20.

As the expanding means 46 is preferably joined at its ends to the substantially non expanding absorbent element 44, it will in turn shorten In longitudinal direction the substantially non expanding absorbent element 44 that will assume the upward concave configuration shown in FIG. 4, with the concave side oriented towards the body facing surface of the sanitary napkin 20.

The extension of the sanitary napkin 20 into a tridimensional structure is therefore achieved in two different steps upon activation by the same amount of fluid; this is particularly advantageous in case of release of a small amount of fluid, since the activation of the shrinkable means 46 helps create a more pronounced expansion of the sanitary napkin 20 into the desired tridimensional structure, e.g. when the expansion of the expanding layer 46 alone may not be sufficient to effectively conform to the user's anatomy.

The expanding layer 46 can also comprise incisions or apertures on at least one of its body facing surface or its garment facing surface, as it is described in European applications EP 96106724.6 and EP 96106723.8 respectively, both applications filed on 29 April 1996, or any combination thereof.

Apertures and incisions, either alone or in combination, improve the swelling capacity of the expanding layer 46 even in localized areas and upon activation by small amounts of fluid, increase the surface area for the acquisition and the diffusion of fluid within the expanding layer 46, and reduce the stiffness of the expanding layer 46 in its dry state.

The use of an expanding layer 46 with apertures therein that involve the whole thickness of the layer in combination with a shrinkable means 48 as illustrated in FIGS. 1 to 4 gives the further advantage of a more effective acquisition of the fluid within the expanding layer 46, and of a more rapid transmission of the fluid through the expanding layer 46 towards the substantially non expanding element 44 and the shrinkable means 48, which therefore can be activated very quickly after the release of the body fluid; this is preferred for a rapid extension of the sanitary napkin 20 into the tridimensional structure by combined activation of both the expanding layer 46 and the shrinkable means 48 at about the same time.

In a preferred embodiment of the present invention the expanding means 46 comprises a sheet of compressed regenerated cellulose sponge.

The regenerated cellulose sponge that preferably constitutes the expanding layer 46 is a material that is known in the art; examples of suitable materials are described in U.S. Patent No. 3,954,493, in French Patent Application FR-A-2,203,827, and in European Patent EP-B-0 293 208. The regenerated cellulose sponge is a sponge of a material containing a cellulose skeleton. Examples of such sponges include, in addition to sponges consisting of cellulose itself, sponges consisting of a cellulose derivative as viscose, a cellulose ether and a cellulose ester, and sponges consisting of mixtures of those materials.

By way of example only, a regenerated cellulose sponge may be prepared from a mixture of a viscose solution with reinforcing fibres and a porogenic compound, e.g. crystals of sodium sulphate decahydrate or of another alkali metal salt with a high content of crystallized water, the final pore dimension being related to that of the salt crystals. The viscose solution may be extruded through an extrusion die of the desired section, then let coagulate. The material is washed with water after regeneration in order to eliminate the salt and other possible soluble compounds, then it is dried and, if necessary, compressed to the desired density.

The compressed regenerated cellulose sponge has a network structure that contains air bubbles created by the elimination of the sodium sulphate crystals.

The compressed regenerated cellulose sponge material is available in various forms, e.g. in layers or sheets of different densities, thicknesses and basis weights; dry densities values for the compressed material used in the present invention are from 0.1 g/cc to 1 g/cc, while thicknesses may range from 0.2 mm to 5 mm.

The swelling upon liquid absorption of the compressed regenerated cellulose sponge material that forms the expanding layer 46 takes place substantially in the direction of the compression and restores the pore size of the regenerated sponge before the compression; it creates a void volume that does not collapse in wet conditions and therefore enables the material to rapidly acquire further releases of fluid and to transmit them to the underlying absorbent element 44 of the absorbent core 28, if present.

In the embodiment illustrated in FIGS. 1 to 4 the total absorbent capacity of the sanitary napkin 20 is provided for by an absorbent core 28 that comprises an expanding layer 46 made of a sheet of compressed regenerated cellulose sponge, and a substantially non expanding absorbent element 44.

One suitable material for the shrinkable means 48 may be a polyvinyl alcohol composition oriented and preferably crosslinked so as to shrink without significant solubility upon contact with body fluids; particularly preferred is the composition produced and marketed by Nitivy Co., Ltd. under the tradename of SOLVRON. SOLVRON is available in form of yarns, continuous filaments, staple fibres or fabric. Useful fabrics may comprise 100% SOLVRON fibres or any mixtures of SOLVRON fibres with other synthetic or natural fibres, for example cotton, nylon, polyethylene therephthalate or polypropylene fibres. A suitable fabric, sold under the tradename of GFX 5788, is made of 100% SX grade, 56 denier SOLVRON fibre; upon contact with body fluids the fabric shows a shrinkage of about 35% both in longitudinal and in transverse directions.

In a preferred embodiment of the present invention a fabric that comprises 25% of the same SOLVRON fibre as described above and 75% of nylon fibres can be used; if polyvinyl alcohol fibres constitute only at least part of the weft of the fabric, while the warp is comprised of nylon alone, the fabric will shrink in the direction of the weft only upon activation by body fluids. The fabric may be accordingly disposed in the sanitary napkin being oriented so as to provide contraction along the desired direction only. A mixed fabric is moreover easier to bond to a substrate, e.g. by known adhesive means or by thermobonding, than a 100% polyvinyl alcohol composition.

In a preferred embodiment of the present invention, which is illustrated in FIGS. 1 and 2, the absorbent core 28 comprises an expanding layer 46 constituted by a sheet of compressed regenerated cellulose sponge with a dry density of 0.5 g/cc and a thickness of 2 mm, positioned on the body facing surface of the absorbent element 44 in face to face relationship with it. Suitable sheets of compressed regenerated cellulose sponge may be those produced by Spontex France.

The shrinkable means 48 comprises a fabric made of 25% SOLVRON fibres and 75% nylon fibres, with the polyvinyl alcohol based fibres longitudinally oriented in order to let the shrinkable means 48 shrink in longitudinal direction only upon activation by body fluids.

The compressed regenerated cellulose sponge sheet that preferably constitutes the expanding layer 46 is capable of absorbing body fluids quickly with a large increase in its volume, generally from about 2 to 20 times, and usually from 5 to 15 times its volume at the time of the compression. The volume increase substantially corresponds to a swelling in the direction of the compression, that is in the Z-direction in the sanitary napkin 20.

The sanitary napkin 20 is produced and packaged as a conventional flat product, as illustrated in FIGS. 1 and 2. After the sanitary napkin 20 has been worn, as soon as the absorbed body fluids come in contact with the expanding layer 46, this will begin to swell in Z-direction increasing its thickness, as can be seen in FIG. 3. The topsheet 24 follows the swelling of the expanding layer 46 by straightening out the pleats or folds 52, therefore increasing its width without restraining the swelling. This is not evident from FIGS. 3 and 4, but is shown in FIG. 6, which illustrates an alternate embodiment of the present invention, with a topsheet 24 folded in the same way as in the embodiment of FIGS. 1 to 4. The further shaping of the sanitary napkin 20 under the action of the shrinking means 48 gives an additional bouncing back effect to the structure of the product when it is subjected to compression in Z-direction during the wearing time; this effect increases the resilience already given to the product by the swollen expanding layer 46.

The swelling of the compressed regenerated cellulose sponge sheet that constitutes the expanding layer 46, combined with the shrinking of the shrinkable means 48, takes place only upon activation by the absorbed fluid, that is only during the use of the sanitary napkin 20 and in close contact with the user's anatomy; the formation of the tridimensional structure can therefore achieve a much better fit with the anatomy of the user. Moreover, the swelling of the compressed regenerated cellulose sponge sheet that constitutes the expanding layer 46 can start where it is actually reached by the fluid first; the formation of the tridimensional structure can also fit, therefore, the different possible ways in which the body fluids may be released by various users.

The expanding topsheet 24, which is a preferred feature of the sanitary napkin 20 of the present invention, also provides a comfortable contact with the user's anatomy, without restraining the expansion of the sanitary napkin 20 into the desired tridimensional structure upon activation by body fluids.

The sanitary napkin 20 of the present invention is flat prior to use, and may be therefore manufactured and packaged more easily than a conventional elasticated or pre-formed article. Since the tridimensional structure is formed only during the use, the sanitary napkin of the present invention is also easier to wear.

In an alternate embodiment of the present invention illustrated in FIGS. 5 and 6 a sanitary napkin 20 similar to that illustrated in FIGS. 1 to 4 comprises a shrinkable means 48 that is capable of shrinking only in transverse direction upon activation by body fluid. FIG. 5 shows a sanitary napkin 20 in which the shrinkable means 48 comprises a rectangular element 50 of a material that is capable of shrinking upon activation by body fluids; the element 50 spans the longitudinal centreline O-O of the sanitary napkin 20 and is generally parallel to the transverse centreline A-A. The length of the element 50 preferably corresponds, as it is illustrated in FIG. 5, to the width of the expanding layer 46, while the width in longitudinal direction is usually smaller. The element 50 is typically centered upon both the longitudinal centreline O-O and the lateral centreline A-A, but it may be positioned differently, or, alternatively, more than one single element 50 may constitute the shrinkable means 48, e.g. two separate shrinkable elements can be used in an embodiment similar to that described in European application EP 96106722.0, filed on 29 April 1996.

The element 50 is joined in any suitable manner, for example stitching or glueing, to the garment facing surface of the expanding layer 46, preferably along its longitudinal side margins that correspond to the longitudinal side margins of the expanding layer 46. The element 50 is also preferably joined along its longitudinal side margins to the body facing surface of the substantially non expanding absorbent element 44.

FIG. 5 shows the sanitary napkin 20 before its extension into a tridimensional structure that is achieved upon activation by body fluid of the expanding means 46 and of the shrinkable means 48. FIG. 6 shows the sanitary napkin 20 after its final extension into the desired tridimensional structure upon activation by a first release of body fluid.

The activation of the expanding layer 46 takes place first, as soon as the body fluid is acquired within the expanding means 4 and causes it to swell, substantially in Z-direction. The second step of the extension of the sanitary napkin 20 into the tridimensional structure corresponds to the activation of the element 50 that constitutes the shrinkable means 48 when the fluid comes into contact with the shrinkable means 48 through the expanding layer 46.

The element 50 of the shrinkable means 48 preferably comprises a material that is capable of shrinking in one direction only, and it is suitably oriented in order to get the shrinkage in lateral direction. As it shrinks it shortens in lateral direction the already partially expanded expanding layer 46, to which it is joined along its longitudinal side margins, and causes it to achieve an arcuate shape with its convex side towards the body facing surface of the sanitary napkin 20, therefore further increasing the extension of the sanitary napkin 20 into the desired tridimensional structure.

The bending of the expanding layer 46 under the action of the shrinkable means 48 that creates the previously described upward convex configuration can make it possible an even better contact between the sanitary napkin 20 and the anatomy of the wearer; moreover, due to the raised profile that is formed, the conformability of the expanding layer 48 to the anatomy is promoted.

As the expanding means 48 is preferably also joined along its longitudinal side margins to the body facing surface of the substantially non expanding absorbent element 44, it will also shorten in lateral direction the substantially non expanding absorbent element 44 as illustrated in FIG. 6; this can help to avoid curls and wrinkles in the backsheet 26.

More than a single shrinkable element 50 can constitute the shrinkable means 48 in alternate embodiments of the present invention, e.g. by combining in a sanitary napkin 20 an expanding layer 46 with two distinct shrinkable elements in such a way that they are each capable of shrinking in a different direction, e.g. one in longitudinal direction and the other in transverse direction; or, alternatively, a single shrinkable element can be used, which is capable of shrinking in both longitudinal and transverse directions. Multiple shrinkable elements, or a single shrinkable element which is shrinkable in two different directions, can be comprised in an absorbent article in combination with the expanding means in different ways that can be easily defined by the man skilled in the art.

An even further embodiment of the present invention is illustrated in FIGS. 7 to 9, in which the combined action of an expanding layer 46 and of a shrinkable means 48 tends to reduce the occurrence of bunching caused in the sanitary napkin 20 by the forces exerted on it during the use, further reducing the risk of fluid leakage from the longitudinal side of the sanitary napkin 20.

FIG. 7 shows a sanitary napkin 20 similar to that illustrated in FIG. 1, with the shrinkable means 48 comprising two rectangular stripes 51 of a shrinkable material that is preferably shrinkable in lateral direction only. Each stripe 51 is joined along its full length to the backsheet 26; its dimensions are such that it spans in length along at least the crotch portion of the sanitary napkin 20, while it is wide enough to be comprised partially between the topsheet 24 and the backsheet 26, and partially between the longitudinal side margins of the substantially non absorbent element 44 and the backsheet 26, as illustrated in FIG. 7.

The forces exerted on the sanitary napkin 20 during the use tend to cause the sanitary napkin 20 to bunch; particularly, the longitudinal side margins of the sanitary napkin 20, that lie very close to the panty profile, tend to bend upside, i.e. towards the wearer's body, and this may also cause part of the released fluid to reach directly the garment facing side of the backsheet 26 and escape laterally.

As soon as the fluid reaches the two stripes 51 from the substantially non expanding absorbent element 44 the material that form the stripes 51 begins to shrink in lateral direction and exerts a force that opposes to the tendency of the longitudinal side margins of the sanitary napkin 20 to bend upward; this effect is illustrated in FIG. 9.

As illustrated in FIG. 7, the pleats or folds 52 of the topsheet 24 are positioned at both sides of the longitudinal centreline O-O and substantially parallel to it, but in each pleat or fold 52 the topsheet 24 is folded twice on itself toward the longitudinal centreline O-O of the sanitary napkin 20. During the swelling of the expanding means 46 upon fluid absorption the straightening out of the pleats or folds 52 forms a sort of longitudinally oriented side cuffs 47 that provide a better seal against side leakage, as illustrated in FIG. 8 and 9; the side cuffs 47 may still be present when the swelling of the expanding layer 46 is completed if the overall width of the topsheet 24 is slightly higher than that which would be necessary to follow the complete swelling of the expanding layer 46.

In an alternate embodiment of the present invention, the sanitary napkin 20 may have two flaps (not shown), each of which are adjacent to and extend laterally from the side edge of the absorbent core. The flaps are configured to drape over the edges of the wearer's panties in the crotch region so that the flaps are disposed between the edges of the wearer's panties and the wearer's thighs. The flaps serve at least two purposes. First, the flaps help serve to prevent soiling of the wearer's body and panties by menstrual fluid, preferably by forming a double wall barrier along the edges of the panty. Second, the flaps are preferably provided with attachment means on their garment facing surface so that the flaps can be folded back under the panty and attached to the garment facing side of the panty. In this way, the flaps serve to keep the sanitary napkin properly positioned in the panty.

The flaps may be constructed of various materials including materials used for the topsheet 24, backsheet 26, combinations thereof, and may be a laminate having tissue in the centre. Further, the flaps may be a separate element attached to the main body of the sanitary napkin 20 or can comprise extensions of the topsheet 24 and/or backsheet 26. It is recommended, however, that the flaps have a liquid impervious backsheet to prevent body fluids which reach the flaps from soiling the edges of the wearer's panties.

Preferred flaps that are suitable or adaptable to the sanitary napkin 20 of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

Optionally, the sanitary napkin 20 may comprise components that naturally wrap the sides of a wearer's panties. Sanitary napkins having components that naturally wrap the sides of a wearer's panties suitable for use with the sanitary napkin 20 of the present invention are disclosed in U.S. Patent Application Serial No. 08/096,121 entitled "Absorbent Article having Panty Covering Components that Naturally Wrap the Sides of Panties", filed July 22, 1993, in the names of Lavash, et al and U.S. Patent Application Serial No. 08/277733 entitled "Absorbent Articles Having Undergarment Covering Components with Zones of Extensibility", filed July 20, 1994, in the names of Weinberger, et al.

The relative positioning of the expanding means and of the shrinkable means 48 in the sanitary napkin 20 can be further varied without departing from the scope of the present invention; e.g., in embodiments similar to those shown in FIGS. 1 to 4 and in FIGS. 5 and 6, the shrinkable means 48 can be comprised between the body facing surface of the expanding layer 46 and the topsheet 24, being joined to the expanding layer 46 only, or, alternatively, the shrinkable means 48 can be joined to the garment facing surface of the substantially non expanding absorbent element 44.

Shrinkable means 48 can also be comprised in different locations in absorbent articles according to the present invention, e.g., shrinkable means in form of yarns or stripes can be comprised within pleats or folds of the topsheet 24 similar to those illustrated in FIGS. 5 and 7; upon activation by body fluids the shrinkable means 48 is capable of raising the pleats or folds thus forming side cuffs for the absorbent article.

The expanding means for expanding the sanitary napkin 20 into a tridimensional structure during wear in combination with the forming or bending due to the shrinkable means 48 can be comprised in the sanitary napkin 20 in any other suitable position and/or orientation in order to get the desired tridimensional structure, in particular it can form at least part of the garment facing surface of the absorbent core 28.

An optional component that can be included in the sanitary napkin 20 of the present invention is an odour control means; any suitable odour control means can be incorporated in the sanitary napkin of the present invention in any desired form, according to the techniques well known in the art.

In a further alternative embodiment (not illustrated) a sanitary napkin 20 similar to that illustrated in FIGS. 1 and 2 can further comprise an acquisition layer or secondary topsheet positioned between the topsheet 24 and the absorbent core 28. Preferably the acquisition layer does not completely overlie the absorbent core 28; e.g. the acquisition layer does not cover the expanding layer 46 that is therefore capable of receiving the body fluids directly through the topsheet 24. Alternatively, the acquisition layer can be comprised between the absorbent core 28 and the backsheet 26; further, the acquisition layer can be comprised between the expanding layer 46 and the shrinkable means 48, and the substantially non expanding absorbent element 44 in an embodiment similar to that illustrated in FIG. 2.

The acquisition layer may serve several functions including improving wicking of body fluids that directly reach the acquisition layer over and into the absorbent core 28. By improving wicking of body fluids, the acquisition layer provides a more even distribution of the body fluids throughout the absorbent core 28.

The acquisition layer preferably comprises materials that are capable of acquiring liquid very fast, and subsequently release it to contiguous layers with substantially no retention capacity.

The acquisition layer may be comprised of several different materials including nonwoven or woven webs of synthetic fibres including polyester, polypropylene, or polyethylene; natural fibres including cotton or cellulose; blends of such fibres; or any equivalent materials or combinations of materials. Examples of sanitary napkins having an acquisition layer and a topsheet are more fully described in U.S. Pat. No. 4,950,264 issued to Osborn and U.S. Pat. Application Serial No. 07/810,774, "Absorbent Article Having Fused Layers", filed December 17, 1991 in the names of Cree, et al.

The topsheet 24, the acquisition layer and the absorbent core 28 may also be associated in any suitable manner, in order to insure proper fluid transfer between them.

In a further alternative embodiment that is not illustrated the acquisition layer may be interposed between the topsheet 24 and the underlying absorbent core 28 comprising the expanding layer 46; the acquisition layer 29 must be left free to follow the expansion of the expanding layer 46 upon absorption of liquid, without restraining its swelling.

Although the disposable absorbent article of the present invention has been described with reference to a sanitary napkin, it can be used beneficially in the context of other disposable absorbent articles such as panty liners, incontinence articles and diapers. The disposable absorbent article may thus also have all those features and parts which are typical for products in the context of their intended use.

## Claims

1. A disposable absorbent article for wearing adjacent a body discharge area, said article having a longitudinal centreline and a lateral centreline orthogonal thereto and defining longitudinal and lateral directions respectively, said article further having a Z-direction which is orthogonal to both said longitudinal and lateral directions, said article comprising a liquid pervious topsheet, a backsheet joined to said topsheet, an absorbent core intermediate said topsheet and said backsheet, said absorbent article being substantially flat prior to use, said absorbent core comprising expanding means for expanding said article into a tridimensional structure while being worn by a user, said means being activated by body fluids, said absorbent core comprising a body facing surface, said absorbent article characterized in that it comprises shrinkable means for further inducing forming or bending of said article in combination with said expanding means, said means being capable of shrinking upon activation by body fluids.

2. A disposable absorbent article according to claim 1, characterized in that said tridimensional structure extends substantially in said Z-direction.

3. A disposable absorbent article according to any preceding claim, characterized in that said expanding means forms at least part of said body facing surface of said absorbent core.

4. A disposable absorbent article according to any preceding claim, characterized in that said shrinkable means is joined to said absorbent core at attachment positions disposed on opposite sides of said lateral centreline and extending longitudinally across, whereby said shrinkable means upon activation by body fluid shortens said disposable absorbent article substantially in longitudinal direction to provide said tridimensional structure in combination with said expanding means.

5. A disposable absorbent article according to any preceding claim, characterized in that said shrinkable means is joined to said absorbent core at attachment positions disposed on opposite sides of said longitudinal centreline and extending laterally across, whereby said shrinkable means upon activation by body fluid shortens said disposable absorbent article substantially in lateral direction to provide said tridimensional structure in combination with said expanding means.

6. A disposable absorbent article according to both claims 4 and 5, characterized in that it comprises two shrinkable means of differing design such that they jointly provide said tridimensional structure in combination with said expanding means.

7. A disposable absorbent article according to any preceding claim, characterized in that said expanding means comprises a compressed regenerated cellulose sponge having a dry density of 0.1÷1 g/cc.

8. A disposable absorbent article according to any preceding claim, characterized in that said shrinkable means comprises a water shrinkable polyvinyl alcohol composition, preferably polyvinyl alcohol fibres.

9. A disposable absorbent article according to any preceding claim, characterized in that said disposable absorbent article is a sanitary napkin or a pantiliner.
